(19) Europäisches Patentamt
European Patent Office
Office européen des brevets

(11) **EP 1 934 165 B1**

(12) **EUROPÄISCHE PATENTSCHRIFT**

(45) Veröffentlichungstag und Bekanntmachung des Hinweises auf die Patenterteilung:
**11.11.2009 Patentblatt 2009/46**

(21) Anmeldenummer: **06793777.1**

(22) Anmeldetag: **25.09.2006**

(51) Int Cl.:
*C07C 209/16* (2006.01)   *C07C 211/10* (2006.01)

(86) Internationale Anmeldenummer:
**PCT/EP2006/066667**

(87) Internationale Veröffentlichungsnummer:
**WO 2007/036498 (05.04.2007 Gazette 2007/14)**

(54) **VERFAHREN ZUR HERSTELLUNG VON ETHYLENAMINEN**

PROCESS FOR PRODUCING ETHYLENEAMINES

PROCEDE DE PRODUCTION D'ETHYLENEAMINES

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **30.09.2005 DE 102005047464**
**06.02.2006 EP 06101332**

(43) Veröffentlichungstag der Anmeldung:
**25.06.2008 Patentblatt 2008/26**

(73) Patentinhaber: **BASF SE**
**67056 Ludwigshafen (DE)**

(72) Erfinder:
• **CAUWENBERGE, Gunther**
**B-9140 Temse (BE)**
• **MELDER, Johann-Peter**
**67459 Böhl-Iggelheim (DE)**
• **EVERS, Holger**
**67063 Ludwigshafen (DE)**
• **GERLACH, Till**
**67071 Ludwigshafen (DE)**
• **KIESSLICH, Frank**
**63128 Dietzenbach (DE)**
• **SCHWAB, Ekkehard**
**67434 Neustadt (DE)**
• **HOFFER, Bram Willem**
**69120 Heidelberg (DE)**

(56) Entgegenhaltungen:
**EP-A- 0 839 575     DE-A1- 19 859 776**
**US-A- 4 014 933**

• **BARNES, CHARLES M. ET AL: "Ethylenediamine by low-pressure ammonolysis of monoethanolamine" INDUSTRIAL & ENGINEERING CHEMISTRY PRODUCT RESEARCH AND DEVELOPMENT, 20(2), 399 -407 CODEN: IEPRA6; ISSN: 0019-7890, 1981, XP002409736 in der Anmeldung erwähnt**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die vorliegende Erfindung betrifft ein Verfahren zur Herstellung von Ethylenaminen durch Umsetzung von Monoethanolamin (MEOA) mit Ammoniak in Gegenwart eines Übergangsmetall-Heterogenkatalysators.

Ethylenamine finden u.a. Verwendung als Lösungsmittel, Stabilisatoren, zur Synthese von Chelat-Bildnern, Kunstharzen, Arzneimitteln, Inhibitoren und grenzflächenaktiven Substanzen.

Insbesondere Diethylentriamin (Bis(2-aminoethyl)amin; DETA) findet Verwendung als Lösungsmittel für Farbstoffe und ist Ausgangsmaterial zur Herstellung von Ionenaustauschern, Schädlingsbekämpfungsmitteln, Antioxidantien, Korrosionsschutzmitteln, Komplexbildnern, Textilhilfsmitteln und Absorptionsmitteln für (saure) Gase.

Zur Herstellung von Ethylenaminen, sind in der Literatur zahlreiche Verfahren beschrieben.

EP 839 575 beschreibt Katalysatoren für die Aminierung von Alkylenoxiden, Alkoholen, Aldehyden und Ketonen.

Gemäß PEP Report No. 138, "Alkyl Amines", SRI International, 03/1981, insbesondere Seiten 7, 8, 13-16, 43-107, 113, 117, liefert die Umsetzung von Dichlorethan mit Ammoniak bei Molverhältnissen von 1 : 15 Diethylentriamin (DETA) mit einem Anteil an den gebildeten Ethylenaminen von größer 20 Gew.-%. Neben 40 Gew.-% Ethylendiamin (EDA) fallen jedoch 40 Gew.-% höhere Ethylenamine an.

Durch Aminierung von Monoethanolamin (MEOA) mit Ammoniak (vgl. z.B. den o.g. PEP Report, US 4,014,933 (BASF AG)) kann die Bildung dieser höheren Ethylenamine (d.h. Ethylenaminen mit einem Siedepunkt über dem von Triethylentetramin (TETA)) zugunsten von Ethylendiamin weitgehend zurückgedrängt werden. Als Nebenprodukte fallen jedoch bei dieser Umsetzung Aminoethylethanolamin (AEEA) und Piperazin (PIP) an.

Ind. Eng. Chem. Prod. Res. Dev. 1981, 20, Seiten 399-407, (C.M. Barnes et al.) beschreibt die Ammonolyse von MEOA zu EDA an Nickel-Katalysatoren auf einem $SiO_2$-$Al_2O_3$-Mischträger. Zusatz von Wasser und der gepulverte Katalysator war angeblich vorteilhaft bei der Erhöhung der Ausbeute an EDA.

Nachteile dieser Technologien ergeben sich bei der Suspensionskatalyse u.a. aus der notwendigen Katalysator/ Produkt Abtrennung. Darüber hinaus sind die Selektivitäten, z.B. für die Bildung von DETA, verbesserungswürdig.

WO-A-05/014523 (BASF AG) betrifft ein Verfahren zur Herstellung von Ethylenaminen (z.B. DETA) durch Umsetzung von Monoethanolamin (MEOA) mit Ammoniak in Gegenwart eines Katalysators in einem Reaktor (1) und Auftrennung des resultierenden Reaktionsaustrags, wobei bei der Auftrennung erhaltenes Ethylendiamin (EDA) in einem separaten Reaktor (2) in Gegenwart eines Katalysators zu Diethylentriamin (DETA) umgesetzt und der resultierende Reaktionsaustrag der Auftrennung des aus Reaktor 1 resultierenden Reaktionsaustrags zugeführt wird.

Die ältere deutsche Patentanmeldung Nr. 102005019373.0 vom 26.04.05 (BASF AG) betrifft ein Verfahren zur Herstellung von Ethylenaminen, in dem man in einer ersten Reaktionsstufe Ethylenoxid mit Ammoniak unter wasserfreien Bedingungen an einem anorganischen Ionenaustauscher kontinuierlich umsetzt, wobei das resultierende Umsetzungsprodukt Monoethanolamin, Diethanolamin und Triethanolamin in einem bestimmten Gewichtsverhältnis enthält, und das Umsetzungsprodukt anschließend kontinuierlich in einer zweiten Reaktionsstufe mit Ammoniak in Gegenwart von Wasserstoff und einem Hydrierkatalysator umsetzt.

Eine parallele deutsche Patentanmeldung mit dem gleichen Anmeldetag (BASF AG) betrifft ein Verfahren zur Herstellung von Ethylenaminen durch Umsetzung von Ethylendiamin (EDA) in Gegenwart spezifischer Heterogenkatalysator-Formkörper.

Eine parallele deutsche Patentanmeldung mit dem gleichen Anmeldetag (BASF AG) betrifft ein Verfahren zur Herstellung von Aminodiglykol (ADG) und Morpholin durch Umsetzung von Diethylenglykol (DEG) mit Ammoniak in Gegenwart spezifischer Heterogenkatalysator-Formkörper.

Der vorliegenden Erfindung lag die Aufgabe zugrunde, den Nachteilen des Stands der Technik abzuhelfen und ein verbessertes wirtschaftliches Verfahren zur Herstellung von Ethylenaminen, wobei es sich bei den Ethylenaminen insbesondere um Ethylendiamin (EDA), Diethylentriamin (DETA), Aminoethylethanolamin (AEEA), Piperazin (PIP), Triethylentetramin (TETA) und/oder höhere lineare Polyethylenamine handelt, aufzufinden.

Das Verfahren soll insbesondere die nicht zyklischen Amine wie Ethylendiamin (EDA) und Diethylentriamin (DETA) in hohen Ausbeuten, Raum-Zeit-Ausbeuten und Selektivitäten liefern.

Z.B. soll der Anteil an Ethylendiamin und Diethylentriamin an den gebildeten Ethylenaminen im Produktmix gegenüber dem Stand der Technik erhöht sein, z.B. größer 70 Gew.-% betragen, und der Anteil an Piperazin (PIP) an den gebildeten Ethylenaminen je nach Bedarf, z.B. auf unter 20 Gew.-%, begrenzt werden können, bei einer Gesamtausbeute bezüglich MEOA von insbesondere größer 70 %.

[Raum-Zeit-Ausbeuten werden angegeben in ‚Produktmenge / (Katalysatorvolumen ● Zeit)' (kg/($l_{Kat.}$ ● h)) und/oder ‚Produktmenge / (Reaktorvolumen ● Zeit)' (kg/($l_{Reaktor}$ ● h))].

Demgemäß wurde ein Verfahren zur Herstellung von Ethylenaminen durch Umsetzung von Monoethanolamin (MEOA) mit Ammoniak in Gegenwart eines Übergangsmetall-Heterogenkatalysators gefunden, welches dadurch gekennzeichnet ist, dass die katalytisch aktive Masse des Katalysators vor der Behandlung mit Wasserstoff sauerstoffhaltige Verbindungen des Aluminiums, Kupfers, Nickels und Kobalts enthält und der Katalysatorformkörper bei Kugelform oder Strangform jeweils einen Durchmesser von < 3 mm, bei Tablettenform eine Höhe von < 3 mm und bei allen anderen Geometrien jeweils einen Äquivalentdurchmesser $L = 1/a'$ von < 0,70 mm aufweist, wobei a' die externe Oberfläche per Volumeneinheit ($mm_s^2/mm_p^3$) ist, mit:

$$a' = \frac{A_p}{V_p},$$

wobei $A_p$ die externe Oberfläche des Katalysatorpartikels ($mm_s^2$) und $V_p$ das Volumen des Katalysatorpartikels ($mm_p^3$) ist.

Die Oberfläche und das Volumen des Katalysatorpartikels (des Katalysatorformkörpers) ergeben sich aus den geometrischen Abmessungen des Partikels (des Formkörpers) gemäß den bekannten mathematischen Formeln.

Das Volumen kann auch nach folgender Methode berechnet werden, bei der man:

1. Die innere Porosität des Formkörpers bestimmt (z.B. über Messung der Wasseraufnahme in [ml/g Kat] bei Raumtemperatur und 1 bar Gesamtdruck),
2. die Verdrängung des Formkörpers beim Eintauchen in eine Flüssigkeit (z.B. durch Gasverdrängung mittels Helium-Pyknometer) bestimmt und
3. die Summe beider Volumina bildet.

Die Oberfläche kann auch nach folgender Methode theoretisch berechnet werden, bei der man eine Umhüllende des Formkörpers definiert, deren Kurvenradien max. 5 μm beträgt (um nicht die innere Porenoberfläche durch "Eindringen" der Umhüllenden in die Poren mitzunehmen) und die den Formkörper möglichst innig berührt (keine Schnittfläche mit dem Träger). Anschaulich würde das einer sehr dünnen Folie entsprechen, die man um den Formkörper legt und dann von innen ein Vakuum anlegt, so dass sich die Folie möglichst eng um den Formkörper legt.

Das als Edukt benötigte Monoethanolamin ($H_2N-CH_2-CH_2-OH$; MEOA) kann nach bekannten Verfahren, beispielsweise durch Umsetzung von Ethylenoxid (EO) mit Ammoniak, hergestellt werden.

Die erfindungsgemäße Umsetzung erfolgt im allgemeinen bei einem Absolutdruck im Bereich von 1-250 bar, bevorzugt 100-220 bar, insbesondere bei 150-200 bar, und im allgemeinen bei erhöhter Temperatur, z.B. im Temperaturbereich von 100-300°C, insbesondere 130-230°C, bevorzugt bei 170-220°C.

MEOA und Ammoniak werden bevorzugt in einem Molverhältnis im Bereich von $NH_3$ : MEOA = 1 - 15, besonders im Bereich von $NH_3$ : MEOA = 4 - 13, ganz besonders im Bereich von $NH_3$ : MEOA = 7 - 11, eingesetzt.

Im allgemeinen werden im erfindungsgemäßen Verfahren die Katalysatoren bevorzugt in Form von Katalysatoren eingesetzt, die entweder nur aus katalytisch aktiver Masse und gegebenenfalls einem Verformungshilfsmittel (wie z.B. Graphit oder Stearinsäure), oder den katalytisch aktiven Komponenten auf einem weitgehend inaktiven Trägermaterial bestehen.

Die katalytisch aktive Masse kann nach Mahlung als Pulver oder als Splitt in das Reaktionsgefäß eingebracht oder bevorzugt, nach Mahlung, Vermischung mit Formhilfsmitteln, Formung und Temperung, als Katalysatorformkörper - beispielsweise als Tabletten, Kugeln, Ringe, Extrudate (z.B. Stränge, Röhren) - in den Reaktor eingebracht werden.

Die Konzentrationsangaben (in Gew.-%) der Komponenten des Katalysators beziehen sich jeweils - falls nicht anders angegeben - auf die katalytisch aktive Masse des hergestellten Katalysators vor der Behandlung mit Wasserstoff.

Die katalytisch aktive Masse des Katalysators ist als die Summe der Massen der katalytisch aktiven Bestandteile definiert und enthält, vor der Behandlung mit Wasserstoff, bevorzugt im Wesentlichen die katalytisch aktiven Bestandteile sauerstoffhaltige Verbindungen des Aluminiums, Kupfers, Nickels und Kobalts.

Die Summe der o.g. katalytisch aktiven Bestandteile, berechnet als $Al_2O_3$, CuO, NiO und CoO, in der katalytisch aktive Masse vor der Behandlung mit Wasserstoff beträgt zum Beispiel 70 bis 100 Gew.-%, bevorzugt 80 bis 100 Gew.-%, besonders bevorzugt 90 bis 100 Gew.-%, insbesondere 95 bis 100 Gew.-%, ganz besonders bevorzugt > 99 bis 100 Gew.-%.

Die sauerstoffhaltigen Verbindungen des Nickels, Kobalts und Kupfers, jeweils berechnet als NiO, CoO und CuO, sind bevorzugt insgesamt in Mengen von 10 bis 80 Gew. %, besonders bevorzugt 15 bis 60 Gew.-%, ganz besonders bevorzugt 20 bis 40 Gew.-%, in der katalytisch aktiven Masse (vor der Behandlung mit Wasserstoff) enthalten, wobei besonders bevorzugt das Molverhältnis von Nickel zu Kupfer größer 1 ist.

Die bevorzugten Katalysatoren im erfindungsgemäßen Verfahren enthalten in ihrer katalytisch aktiven Masse vor der Behandlung mit Wasserstoff

20 bis 90 Gew.-%, bevorzugt 40 bis 85 Gew.-%, besonders bevorzugt 60 bis

80 Gew.-%, sauerstoffhaltige Verbindungen des Aluminiums, berechnet als $Al_2O_3$,

1 bis 30 Gew.-%, bevorzugt 2 bis 25 Gew.-%, besonders bevorzugt 3 bis 20 Gew.-%, sauerstoffhaltige Verbindungen des Kupfers, berechnet als $CuO$,

1 bis 40 Gew.-%, bevorzugt 3 bis 30 Gew.-%, besonders bevorzugt 5 bis 20 Gew.-%, sauerstoffhaltige Verbindungen des Nickels, berechnet als $NiO$, wobei besonders bevorzugt das Molverhältnis von Nickel zu Kupfer größer 1, bevorzugt größer 1,2, besonders bevorzugt 1,8 bis 8,5, ist, und

1 bis 40 Gew.-%, bevorzugt 3 bis 30 Gew.-%, besonders bevorzugt 5 bis 20 Gew.-%, sauerstoffhaltige Verbindungen des Kobalts, berechnet als $CoO$.

Zur Herstellung der Katalysatoren mit der genannten Zusammensetzung sind verschiedene Verfahrensweisen möglich. Sie sind zum Beispiel durch dem Fachmann bekannte Fällungsverfahren und bevorzugt Tränkungsverfahren erhältlich.

Besonders bevorzugte Katalysatoren im erfindungsgemäßen Verfahren sind die in DE-A-19 53 263 (BASF AG) offenbarten Katalysatoren enthaltend Kobalt, Nickel und Kupfer und Aluminiumoxid und optional Siliciumdioxid mit einem Metallgehalt von 5 bis 80 Gew.-%, insbesondere 10 bis 30 Gew.-%, bezogen auf den gesamten Katalysator, wobei die Katalysatoren, berechnet auf den Metallgehalt, 70 bis 95 Gew.-% einer Mischung aus Kobalt und Nickel und 5 bis 30 Gew.-% Kupfer enthalten und wobei das Gewichtsverhältnis von Kobalt zu Nickel 4 : 1 bis 1 : 4, insbesondere 2 : 1 bis 1 : 2, beträgt, beispielsweise der in den dortigen Beispielen verwendete Katalysator mit der Zusammensetzung 10 Gew.-% CoO, 10 Gew.-% NiO und 4 Gew.-% CuO auf $Al_2O_3$.

Der eingesetzte Katalysator weist bevorzugt eine Schüttdichte im Bereich von 0,6 bis 1,2 kg/l auf.

Erfindungsgemäß wurde erkannt, dass besonders hohe EDA- und DETA-Selektivitäten erhalten werden, wenn der Katalysator in Form kleiner Formkörper eingesetzt wird. Mit kleinem Formkörper sind solche gemeint, deren Durchmesser bei Kugelform jeweils unterhalb von 3 mm, insbesondere unterhalb von 2,5 mm, z.B. im Bereich von 1 bis 2 mm, liegt.

Entsprechend sind mit kleinem Formkörper auch solche gemeint, deren Durchmesser bei Strangform (Stranglänge >> Strangdurchmesser) oder deren Höhe bei Tablettenform (Tablettendurchmesser >> Tablettenhöhe) jeweils unterhalb von 3 mm, insbesondere unterhalb von 2,5 mm, z.B. im Bereich von 1 bis 2 mm, liegt.

Bei allen anderen Geometrien weist der Katalysatorformkörper im erfindungsgemäßen Verfahren jeweils einen Äquivalentdurchmesser L = 1/a' von < 0,70 mm, insbesondere von < 0,65 mm, z.B. im Bereich von 0,2 bis 0,6 mm, auf, wobei a' die externe Oberfläche per Volumeneinheit ($mm_s^2/mm_p^3$) ist, mit:

$$a' = \frac{A_p}{V_p},$$

wobei $A_p$ die externe Oberfläche des Katalysatorpartikels ($mm_s^2$) und $V_p$ das Volumen des Katalysatorpartikels ($mm_p^3$) ist. (L = Spezifische Dimension eines Katalysatorformköpers).

Im erfindungsgemäßen Verfahren sind durch die kleine spezifische Dimension der Katalysatorpartikel die Diffusionswege der Reaktanden und auch der Produkte geringer. Die mittlere Verweilzeit der Moleküle in den Poren und die Wahrscheinlichkeit einer unerwünschten Folgereaktion werden hierdurch herabgesetzt. Als Folge der definierten Verweilzeit kann damit eine erhöhte Selektivität, insbesondere in Richtung des erwünschten EDAs und DETAs, erreicht werden.

Bevorzugt liegt der Katalysator als Festbett in einem Reaktor vor. Bei dem Reaktor handelt es sich bevorzugt um einen Rohrreaktor oder Rohrbündelreaktor. Bevorzugt erfolgt die Umsetzung von MEOA im Reaktor im geraden Durchgang.

Das Katalysatorbett wird bevorzugt sowohl am Eingang als am Ausgang des Reaktors von einem Inertmaterial umgeben. Als Inertmaterial können z.B. Pallringe, Kugeln aus einem inerten Material (z.B. Keramik, Steatit, Aluminium) eingesetzt werden.

Der Reaktor kann sowohl in Sumpf- als in Rieselfahrweise betrieben werden. Bei der bevorzugten Rieselfahrweise wird bevorzugt ein Flüssigkeitsverteiler für den Reaktorfeed am Eingang des Reaktors eingesetzt.

Zur Aufrechterhaltung der Katalysatoraktivität werden bevorzugt 0,01 - 1,00 Gew.-%, besonders 0,20 - 0,60 Gew.-%, Wasserstoff (bezogen auf den Reaktorfeed MEOA + $NH_3$) in den Reaktor gefahren.

Im bevorzugt kontinuierlichen Betrieb werden bei einer WHSV (weight hourly space velocity) von 0,25 - 1,25 kg/kg*h, bevorzugt 0,4 - 1 kg/kg*h (kg MEOA pro kg Kat. pro Stunde) im Umsatzbereich von 55 - 90 % Selektivitäten (S) bezüglich EDA und DETA von bevorzugt ≥ 60 %, insbesondere 70 - 85 %, erreicht.

Als weitere Produkte fallen im erfindungsgemäßen Verfahren geringe Mengen an Piperazin ($S_{PIP}$ im allgemeinen 5 - 25 %), Aminoethylethanolamin ($S_{AEEA}$ im allgemeinen 3 - 13 %) und höhere Amine ($S_{höhere\ Amine}$ im allgemeinen 2 - 12 %) an.

Im Allgemeinen enthalten die Reaktionsrohprodukte des erfindungsgemäßen Verfahrens nur geringe Mengen an zyklische Aminen als Reaktionsprodukte (in der Regel in Mengen < 20 Gew.-%, insbesondere < 15 Gew.-%, ganz besonders 7 bis 12 Gew.-%).

Im Allgemeinen enthalten die Reaktionsrohprodukte des erfindungsgemäßen Verfahrens nur geringe Mengen an tertiären Aminen als Reaktionsnebenprodukte (in der Regel in Mengen < 10 Gew.-%, insbesondere < 7 Gew.-%, ganz besonders 0 bis 4 Gew.-%).

Die Aufarbeitung der im erfindungsgemäßen Verfahren anfallenden Produktströme, die vor allem das besonders gewünschte EDA und DETA, aber auch N-(2-Aminoethyl)-ethanolamin (AEEA), Triethylentetramin (TETA), Piperazin (PIP), N-(2-Aminoethyl)-piperazin (AE-PIP) und unumgesetztes MEOA enthalten, kann nach dem Fachmann bekannten Destillationsverfahren erfolgen. (Vergl. z.B. PEP Report No. 138, "Alkyl Amines", SRI International, 03/1981, Seiten 81-99, 117, und DE-A-10349059 (BASF-AG)).

Die zur destillativen Reingewinnung der einzelnen Produkte, vor allem des besonders gewünschten EDAs und DETAs, benötigten Destillationskolonnen können durch den Fachmann mit ihm geläufigen Methoden ausgelegt werden (z.B. Zahl der Trennstufen, Rücklaufverhältnis, etc.).

Die Auftrennung des aus der Umsetzung resultierenden Reaktionsaustrags erfolgt insbesondere durch mehrstufige Destillation.

Zum Beispiel erfolgt die Auftrennung des aus der Umsetzung resultierenden Reaktionsaustrags in zwei Trennsequenzen durch mehrstufige Destillation, wobei in der ersten Trennsequenz zunächst Ammoniak und gegebenenfalls vorhandener Wasserstoff abgetrennt werden und in der zweiten Trennsequenz eine Auftrennung in unumgesetztes MEOA sowie EDA, PIP, DETA, AEEA, AE-PIP, TETA und höhere Ethylenamine erfolgt.

Bei der Auftrennung des aus der Umsetzung resultierenden Reaktionsaustrags anfallender Ammoniak und/ oder anfallendes MEOA werden bevorzugt in die Umsetzung zurückgeführt.

**Beispiele**

Folgende Katalysatoren wurden zur Umsetzung von MEOA mit Ammoniak zu Ethylenaminen eingesetzt:

Alle Katalysatoren waren Cu/Co/Ni/gamma-Al$_2$O$_3$ - Katalysatoren wie z.B. in DE-A-19 53 263 (BASF AG) offenbart und durch Tränkung hergestellt.

Die Katalysatoren 1-3 wiesen folgende Zusammensetzung vor ihrer Behandlung (Aktivierung) mit Wasserstoff auf:

10 Gew.-% CoO, 10 Gew.-% NiO und 4 Gew.-% CuO auf gamma-Al$_2$O$_3$.
Katalysator 1 lag in Form von Strängen (Durchmesser D = 4 mm) vor.
Katalysator 2 lag in Form von Strängen (Durchmesser D = 2,5 mm) vor.
Katalysator 3 wurde durch entsprechendes Tränken eines gamma-Al$_2$O$_3$-Stranges mit 1,5 mm Durchmesser erhalten.

| Beispiel | D[1] / mm | L[2] / mm |
|---|---|---|
| 1 (Vergleich) | 4 | 6,5 |
| 2 | 2,5 | 6 |
| 3 | 1,5 | 6,9 |
| [1] mittlerer Strangdurchmesser [2] mittlere Stranglänge | | |

Beispiele 1 bis 3:

| | D(Strang) | Temperatur | Druck | Belastung | Umsatz | S(EDA+DETA) |
|---|---|---|---|---|---|---|
| | mm | °C | bar | kg/l•h | % | % |
| 1 (Vergleich) | 4 | 174,8 | 202,7 | 0,54 | 64,0 | 71,9 |

(fortgesetzt)

|  | D(Strang) | Temperatur | Druck | Belastung | Umsatz | S(EDA+DETA) |
|---|---|---|---|---|---|---|
|  | mm | °C | bar | kg/l•h | % | % |
| 2 | 2,5 | 175,0 | 220,0 | 0,55 | 65,0 | 76,0 |
| 3 | 1,5 | 175,0 | 200,0 | 0,55 | 66,0 | 80,5 |

Aus den Beispielen wird ersichtlich, dass bei sonst nahezu identischen Versuchsbedingungen mit sinkender Größe des Formkörpers erhöhte Umsätze Richtung EDA und DETA realisiert werden.

**Patentansprüche**

1. Verfahren zur Herstellung von Ethylenaminen durch Umsetzung von Monoethanolamin (MEOA) mit Ammoniak in Gegenwart eines Übergangsmetall-Heterogenkatalysators, **dadurch gekennzeichnet, dass** die katalytisch aktive Masse des Katalysators vor der Behandlung mit Wasserstoff sauerstoffhaltige Verbindungen des Aluminiums, Kupfers, Nickels und Kobalts enthält und der Katalysatorformkörper bei Kugelform oder Strangform jeweils einen Durchmesser von < 3 mm, bei Tablettenform eine Höhe von < 3 mm und bei allen anderen Geometrien jeweils einen Äquivalentdurchmesser $L = 1/a'$ von < 0,70 mm aufweist, wobei $a'$ die externe Oberfläche per Volumeneinheit ($mm_s^2/mm_p^3$) ist, mit:

$$a' = \frac{A_p}{V_p},$$

wobei $A_p$ die externe Oberfläche des Katalysatorpartikels ($mm_s^2$) und $V_p$ das Volumen des Katalysatorpartikels ($mm_p^3$) ist.

2. Verfahren zur Herstellung von Ethylenaminen nach Anspruch 1, wobei es sich bei den Ethylenaminen um Ethylendiamin (EDA), Diethylentriamin (DETA), Aminoethylethanolamin (AEEA), Piperazin (PIP), N-(2-Aminoethyl)-piperazin (AE-PIP) und/oder Triethylentetramin (TETA) handelt.

3. Verfahren zur Herstellung von Ethylenaminen nach Anspruch 1 oder 2, wobei es sich bei den Ethylenaminen um nicht zyklische Ethylenamine handelt.

4. Verfahren zur Herstellung von Ethylenaminen nach einem der vorhergehenden Ansprüche, wobei es sich bei den Ethylenaminen um Ethylendiamin (EDA) und Diethylentriamin (DETA) handelt.

5. Verfahren nach einem der vorhergehenden Ansprüche, **dadurch gekennzeichnet, dass** der Katalysatorformkörper bei Kugelform oder Strangform jeweils einen Durchmesser von < 2,5 mm, bei Tablettenform eine Höhe von < 2,5 mm und bei allen anderen Geometrien jeweils einen Äquivalentdurchmesser $L = 1/a'$ von < 0,65 mm aufweist.

6. Verfahren nach einem der vorhergehenden Ansprüche, **dadurch gekennzeichnet, dass** die Umsetzung von MEOA in Gegenwart von Wasserstoff durchgeführt wird.

7. Verfahren nach einem der vorhergehenden Ansprüche, **dadurch gekennzeichnet, dass** die Umsetzung von MEOA bei einer Temperatur im Bereich von 100 bis 300°C durchgeführt wird.

8. Verfahren nach einem der vorhergehenden Ansprüche, **dadurch gekennzeichnet, dass** die Umsetzung von MEOA bei einem Absolutdruck im Bereich von 1 bis 250 bar durchgeführt wird.

9. Verfahren nach einem der vorhergehenden Ansprüche, **dadurch gekennzeichnet, dass** die Umsetzung von MEOA in der Gasphase, Flüssigphase oder in überkritischer Phase durchgeführt wird.

10. Verfahren nach einem der vorhergehenden Ansprüche, **dadurch gekennzeichnet, dass** die katalytisch aktive Masse des Katalysators vor der Behandlung mit Wasserstoff
20 bis 90 Gew.-% sauerstoffhaltige Verbindungen des Aluminiums, berechnet als $Al_2O_3$,
1 bis 30 Gew.-% sauerstoffhaltige Verbindungen des Kupfers, berechnet als CuO,
1 bis 40 Gew.-% sauerstoffhaltige Verbindungen des Nickels, berechnet als NiO und
1 bis 40 Gew.-% sauerstoffhaltige Verbindungen des Kobalts, berechnet als CoO, enthält.

**11.** Verfahren nach einem der vorhergehenden Ansprüche, **dadurch gekennzeichnet, dass** der eingesetzte Katalysator eine Schüttdichte im Bereich von 0,6 bis 1,2 kg/l aufweist.

**12.** Verfahren nach einem der vorhergehenden Ansprüche, **dadurch gekennzeichnet, dass** der Katalysator als Festbett in einem Reaktor vorliegt.

**13.** Verfahren nach dem vorhergehenden Anspruch, **dadurch gekennzeichnet, dass** es sich bei dem Reaktor um einen Rohrreaktor oder Rohrbündelreaktor handelt.

**14.** Verfahren nach einem der beiden vorhergehenden Ansprüche, **dadurch gekennzeichnet, dass** die Umsetzung von MEOA im Reaktor im geraden Durchgang erfolgt.

**15.** Verfahren nach einem der drei vorhergehenden Ansprüche, **dadurch gekennzeichnet, dass** der Reaktor in Sumpffahrweise oder in Rieselfahrweise betrieben wird.

**16.** Verfahren nach einem der vorhergehenden Ansprüche, **dadurch gekennzeichnet, dass** man MEOA und Ammoniak in einem Molverhältnis im Bereich von $NH_3$ : MEOA = 1 - 15 einsetzt.

**17.** Verfahren nach einem der vorhergehenden Ansprüche, **dadurch gekennzeichnet, dass** die Auftrennung des aus der Umsetzung resultierenden Reaktionsaustrags durch mehrstufige Destillation erfolgt.

**18.** Verfahren nach dem vorhergehenden Anspruch, **dadurch gekennzeichnet, dass** die Auftrennung des aus der Umsetzung resultierenden Reaktionsaustrags in zwei Trennsequenzen durch mehrstufige Destillation erfolgt, wobei in der ersten Trennsequenz zunächst Ammoniak und gegebenenfalls vorhandener Wasserstoff abgetrennt werden und in der zweiten Trennsequenz eine Auftrennung in unumgesetztes MEOA, EDA, PIP, DETA, AEEA, AE-PIP, TETA und höhere Ethylenamine erfolgt.

**19.** Verfahren nach einem der vorhergehenden Ansprüche, **dadurch gekennzeichnet, dass** bei der Auftrennung des aus der Umsetzung resultierenden Reaktionsaustrags anfallender Ammoniak und/oder anfallendes MEOA in die Umsetzung zurückgeführt wird.

**Claims**

**1.** A process for preparing ethylene amines by reaction of monoethanolamine (MEOA) with ammonia in the presence of a heterogeneous transition metal catalyst, wherein the catalytically active composition of the catalyst before treatment with hydrogen comprises oxygen-comprising compounds of aluminum, copper, nickel and cobalt and the shaped catalyst body has a diameter of < 3 mm in the case of a spherical shape or extrudate form, a height of < 3 mm in the case of a pellet shape and in the case of all other geometries in each case an equivalent diameter L = 1/a' of < 0.70 mm, where a' is the external surface area per unit volume ($mm_s^2/mm_p^3$), with:

$$a' = \frac{A_p}{V_p},$$

where $A_p$ is the external surface area of the catalyst particle ($mm_s^2$) and $V_p$ is the volume of the catalyst particle ($mm_p^3$).

**2.** The process for preparing ethylene amines according to claim 1, wherein the ethylene amines are ethylenediamine (EDA), diethylenetriamine (DETA), aminoethylethanolamine (AEEA), piperazine (PIP), N-(2-aminoethyl)piperazine (AE-PIP) and/or triethylenetetramine (TETA).

3. The process for preparing ethylene amines according to claim 1 or 2, wherein the ethylene amines are acyclic ethylene amines.

4. The process for preparing ethylene amines according to any of the preceding claims, wherein the ethylene amines are ethylenediamine (EDA) and diethylenetriamine (DETA).

5. The process according to any of the preceding claims, wherein the shaped catalyst body has a diameter of < 2.5 mm in the case of a spherical shape or extrudate form, a height of < 2.5 mm in the case of a pellet shape and in the case of all other geometries in each case an equivalent diameter $L = 1/a'$ of < 0.65 mm.

6. The process according to any of the preceding claims, wherein the reaction of MEOA is carried out in the presence of hydrogen.

7. The process according to any of the preceding claims, wherein the reaction of MEOA is carried out at a temperature in the range from 100 to 300°C.

8. The process according to any of the preceding claims, wherein the reaction of MEOA is carried out at an absolute pressure in the range from 1 to 250 bar.

9. The process according to any of the preceding claims, wherein the reaction of MEOA is carried out in the gas phase, the liquid phase or in the supercritical phase.

10. The process according to any of the preceding claims, wherein the catalytically active composition of the catalyst before treatment with hydrogen comprises
from 20 to 90% by weight of oxygen-comprising compounds of aluminum, calculated as $Al_2O_3$,
from 1 to 30% by weight of oxygen-comprising compounds of copper, calculated as CuO,
from 1 to 40% by weight of oxygen-comprising compounds of nickel, calculated as NiO, and
from 1 to 40% by weight of oxygen-comprising compounds of cobalt, calculated as CoO.

11. The process according to any of the preceding claims, wherein the catalyst used has a bulk density in the range from 0.6 to 1.2 kg/l.

12. The process according to any of the preceding claims, wherein the catalyst is present as a fixed bed in a reactor.

13. The process according to the preceding claim, wherein the reactor is a tube reactor or a shell-and-tube reactor.

14. The process according to either of the two preceding claims, wherein the reaction of MEOA is carried out in a single pass through the reactor.

15. The process according to any of the three preceding claims, wherein the reactor is operated in the upflow mode or in the downflow mode.

16. The process according to any of the preceding claims, wherein MEOA and ammonia are used in a molar ratio in the range $NH_3$:MEOA = 1-15.

17. The process according to any of the preceding claims, wherein the fractionation of the reaction product mixture resulting from the reaction is carried out by multistage distillation.

18. The process according to the preceding claim, wherein the fractionation of the reaction product mixture resulting from the reaction is carried out by multistage distillation in two separation sequences, with ammonia and any hydrogen present being separated off first in the first separation sequence and fractionation into unreacted MEOA, EDA, PIP, DETA, AEEA, AE-PIP, TETA and higher ethylene amines being carried out in the second separation sequence.

19. The process according to any of the preceding claims, wherein the ammonia obtained from the reaction product mixture resulting from the reaction from the fractionation and/or MEOA obtained are/is recirculated to the reaction.

**Revendications**

1. Procédé de fabrication d'éthylèneamines par réaction de monoéthanolamine (MEOA) avec de l'ammoniac en présence d'un catalyseur hétérogène à base d'un métal de transition, **caractérisé en ce que** la masse catalytiquement active du catalyseur contient, avant le traitement avec de l'hydrogène, des composés oxygénés d'aluminium, de cuivre, de nickel et de cobalt et le corps moulé du catalyseur présente, lorsqu'il est sous la forme d'une bille ou sous la forme d'un filament, à chaque fois un diamètre < 3 mm, lorsqu'il est sous la forme d'une tablette, une hauteur < 3 mm, et lorsqu'il a une autre géométrie, à chaque fois un diamètre équivalent L = 1/a' < 0,70 mm, a' étant la surface externe par unité de volume ($mm_s^2/mm_p^3$), avec

$$a' = \frac{A_p}{V_p},$$

$A_p$ représentant la surface externe de la particule de catalyseur ($mm_s^2$) et $V_p$ le volume de la particule de catalyseur ($mm_p^3$).

2. Procédé de fabrication d'éthylèneamines selon la revendication 1, dans lequel les éthylèneamines sont l'éthylènediamine (EDA), la diéthylènetriamine (DETA), l'aminoéthyléthanolamine (AEEA), la pipérazine (PIP), la N-(2-aminoéthyl)-pipérazine (AE-PIP) et/ou la triéthylènetétramine (TETA).

3. Procédé de fabrication d'éthylèneamines selon la revendication 1 ou 2, dans lequel les éthylèneamines sont des éthylèneamines non cycliques.

4. Procédé de fabrication d'éthylèneamines selon l'une quelconque des revendications précédentes, dans lequel les éthylèneamines sont l'éthylènediamine (EDA) et la diéthylènetriamine (DETA).

5. Procédé selon l'une quelconque des revendications précédentes, **caractérisé en ce que** le corps moulé du catalyseur présente, lorsqu'il est sous la forme d'une bille ou sous la forme d'un filament, à chaque fois un diamètre < 2,5 mm, lorsqu'il est sous la forme d'une tablette, une hauteur < 2,5 mm, et lorsqu'il a une autre géométrie, à chaque fois un diamètre équivalent L = 1/a' < 0,65 mm.

6. Procédé selon l'une quelconque des revendications précédentes, **caractérisé en ce que** la réaction de la MEOA est réalisée en présence d'hydrogène.

7. Procédé selon l'une quelconque des revendications précédentes, **caractérisé en ce que** la réaction de la MEOA est réalisée à une température dans la plage allant de 100 à 300 °C.

8. Procédé selon l'une quelconque des revendications précédentes, **caractérisé en ce que** la réaction de la MEOA est réalisée à une pression absolue dans la plage allant de 1 à 250 bars.

9. Procédé selon l'une quelconque des revendications précédentes, **caractérisé en ce que** la réaction de la MEOA est réalisée dans la phase gazeuse, la phase liquide ou dans la phase surcritique.

10. Procédé selon l'une quelconque des revendications précédentes, **caractérisé en ce qu'**avant le traitement avec de l'hydrogène, la masse catalytiquement active du catalyseur contient
    20 à 90 % en poids de composés oxygénés d'aluminium, calculé sous la forme $Al_2O_3$,
    1 à 30 % en poids de composés oxygénés de cuivre, calculé sous la forme CuO,
    1 à 40 % en poids de composés oxygénés de nickel, calculé sous la forme NiO et
    1 à 40 % en poids de composés oxygénés de cobalt, calculé sous la forme CoO.

11. Procédé selon l'une quelconque des revendications précédentes, **caractérisé en ce que** le catalyseur utilisé présente une densité apparente dans la plage allant de 0,6 à 1,2 kg/l.

12. Procédé selon l'une quelconque des revendications précédentes, **caractérisé en ce que** le catalyseur se présente sous la forme d'un lit solide dans un réacteur.

**13.** Procédé selon la revendication précédente, **caractérisé en ce que** le réacteur est un réacteur tubulaire ou un réacteur à faisceau de tubes.

**14.** Procédé selon l'une quelconque des deux revendications précédentes, **caractérisé en ce que** la réaction de la MEOA a lieu dans le réacteur en passage direct.

**15.** Procédé selon l'une quelconque des trois revendications précédentes, **caractérisé en ce que** le réacteur est utilisé en mode de fond ou en mode de ruissellement.

**16.** Procédé selon l'une quelconque des revendications précédentes, **caractérisé en ce que** la MEOA et l'ammoniac sont utilisés en un rapport molaire dans la plage NH$_3$ : MEOA = 1-15.

**17.** Procédé selon l'une quelconque des revendications précédentes, **caractérisé en ce que** la séparation de la sortie de réaction résultant de la réaction a lieu par une distillation à plusieurs étapes.

**18.** Procédé selon la revendication précédente, **caractérisé en ce que** la séparation de la sortie de réaction résultant de la réaction a lieu en deux séquences de séparation par une distillation à plusieurs étapes, l'ammoniac et éventuellement l'hydrogène présent étant tout d'abord séparés lors de la première séquence de séparation, puis, lors de la seconde séquence de séparation, une séparation en MEOA non réagie, EDA, PIP, DETA, AEEA, AE-PIP, TETA et éthylèneamines supérieures ayant lieu.

**19.** Procédé selon l'une quelconque des revendications précédentes, **caractérisé en ce que** lors de la séparation de la sortie de réaction résultant de la réaction, l'ammoniac formé et/ou le MEOA formé est recyclé dans la réaction.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 839575 A
- US 4014933 A
- WO 05014523 A

- DE 102005019373
- DE 1953263 A
- DE 10349059 A

**In der Beschreibung aufgeführte Nicht-Patentliteratur**

- **C.M. Barnes.** *Ind. Eng. Chem. Prod. Res. Dev.,* 1981, vol. 20, 399-407

- Vergl. z.B. PEP Report No. 138, ''Alkyl Amines''. *SRI International,* Marz 1981, 81-99117